# EUROPEAN PATENT APPLICATION

(11) **EP 4 379 037 A2**
(43) Date of publication of application: **05.06.2024**
(21) Application number: 23212403.2
(22) Date of filing: 27.11.2023
(51) Int. Cl.: C12M 1/00

(54) **IMPROVEMENTS IN OR RELATING TO CELLULAR ASSAYS**

(30) Priority: 29.11.2022 GB 202217896
(71) Applicant: 3Brain AG, 8808 Pfäffikon SZ (CH)
(72) Inventor: MACCIONE,, Alessandro, 8808 Pfäffikon SZ (CH); GANDOLFO,, Mauro, 8808 Pfäffikon SZ (CH); IMFELD,, Kilian, 8808 Pfäffikon SZ (CH); ISCHER,, Réal, 2002 Neuchâtel (CH); LEDROIT,, Diane, 2002 Neuchâtel (CH); HEUB,, Sarah, 2002 Neuchâtel (CH)
(74) Representative: Potter Clarkson

(57) **Abstract**

In the field of cell-based assays, i.e. cellular assays, there is a need for improved cell culture retaining inserts, cell culture retaining insert assemblies, cell culture positioning inserts, and cell culture positioning insert assemblies.

A cell culture retaining insert (10; 50; 70), for retaining a three-dimensional biological sample (56) in a cell culture vessel (122; 132) comprises a rigid insert body (12) which has at least one engagement formation (14) to, in use, removably secure the cell culture retaining insert (10; 50; 70) to the cell culture vessel (122, 132). The cell culture retaining insert (10; 50; 70) also includes a retention element (18; 18'; 18") that is coupled with the insert body (12). The retention element (18; 18'; 18") has a distal end (28) that is positionable, in use, proximate to a well bottom (90) in a cell culture vessel (122; 132). The entire distal end (28) is resiliently deformable.

## Description

This invention relates to a cell culture retaining insert, a cell culture retaining insert assembly, a cell culture positioning insert, and cell culture positioning insert assembly, and a cellular assay including such inserts and insert assemblies.

Cell-based assays, i.e. cellular assays, are important experimental tools in life science research and biomanufacturing. They may be based on cell culture methods, where live cells are grown in vitro and used as model systems to assess the biochemistry and physiology of both healthy and diseased cells. Such three-dimensional biological samples may also contain one or more non-living cells, or be or include a micro-tissue, a spheroid, an organoid, a tumoroid, an assembloid or a biopsy. They may also include a gel bead which contains a single cell or multiple cells within its volume.

According to a first aspect of the invention there is provided a cell culture retaining insert, for retaining a three-dimensional biological sample in a cell culture vessel, the cell culture retaining insert comprising:
a rigid insert body having at least one engagement formation to in-use removably secure the cell culture retaining insert to a cell culture vessel; and
a retention element coupled with the insert body, the retention element having a distal end positionable in-use proximate to a well bottom in a cell culture vessel, the entire distal end being resiliently deformable.

Having a distal end which is resiliently deformable allows the retention element to accommodate the varying shape of one or more three-dimensional biological samples while urging the or each sample into abutment with a well bottom within a cell culture vessel, and thereby permits the desired retention of the or each sample within the associated well of the cell culture vessel without damaging the respective sample. Such retention of the or each sample advantageously immobilises it within the well and allows the cell culture vessel to be safely moved without the sample being dislodged.

In addition, having a distal end, the entirety of which is resiliently deformable, beneficially allows any part of the distal end to accommodate the or each sample, and thus permits the ready and reliable retention of samples irrespective of where they are positioned in a well bottom, i.e. such samples do not need to be perfectly positioned in the centre of a well bottom to be readily and reliably retained by the cell culture retaining insert of the invention.

A still further benefit of having an entire distal end which is resiliently deformable is that, as well as being able to accommodate varying shapes of one or more three-dimensional biological samples positioned anywhere in the well bottom of a corresponding well of a cell culture vessel, such a distal end is also able to accommodate varying shapes of well, e.g. those with a flat bottom or a round well bottom, along with those cells having a microstructured bottom, i.e. a well bottom which includes one or more formations extending from or being recessed into an otherwise uniform surface.

Preferably the distal end of the retention element is or includes a planar retention surface having one or more retention walls depending therefrom.

Such a combination of retention surface and one or more retention walls desirably provides a necessary degree of flexibility to accommodate a given sample, while nevertheless having a required degree of structural integrity to continue to urge the sample towards a well bottom and thereby ensure the sample remains retained in position within the associated well.

Optionally the planar retention surface includes a plurality of conduits passing therethrough.

Including a plurality of conduits in the planar retention surface safely facilitates the selective introduction and removal of a liquid, e.g. an aqueous-based buffer, cell culture medium, or hydrogel, to/from contact with a given sample, while ensuring the sample remains retained in position within a well of a cell culture vessel.

Such a plurality of conduits also desirably permits a user of the retaining insert of the invention, e.g. a laboratory technician or other scientist, to visually inspect or observe a given biological sample, either with the naked eye or under a microscope, while the said sample nevertheless similarly remains retained in position.

At least one retention wall may include one or more fluid conduits passing therethrough.

Including one or more fluid conduits in at least one retention wall facilitates the selective introduction and removal of a liquid to/from contact with the sample, while ensuring the sample remains retained in position within a well of a cell culture vessel. In a preferred embodiment of the invention at least one retention wall includes a tapered portion.

The inclusion of one or more such tapered retention wall portions within the retention element allows for the provision of various retention elements, each of which provides a different-sized retention surface, but all of which are able to couple with the insert body in the same manner. Thus, different-sized distal ends can be provided, e.g. according to the nature and/or size of the sample to be retained, the geometry or other spatial features of a corresponding well of a cell culture vessel, and/or the nature of the sample investigation to be carried out, without having to modify the insert body.

In another preferred embodiment of the invention at least one retention wall includes one or more areas having a reduced cross-sectional thickness.

The inclusion in at least one retention wall of one or more areas having a reduced cross-sectional thickness, provides the option of tuning the retention force ultimately applied by the retention surface from which the or each such retention wall depends.

Preferably the planar retention surface is substantially flat.

Having a substantially flat planar retention surface helps to ensure that a substantially uniform retention force is applied across the majority of the retention surface.

The planar retention surface may have a convex or a concave cross-sectional profile.

Providing the planar retention surface with a convex or concave cross-sectional profile advantageously modifies the retention force profile applied to a sample retained thereby, with the convex cross-sectional profile applying an increasingly greater retention force towards the centre of the planar retention surface and the concave cross-sectional profile applying an increasing lower retention force towards the centre of the planar retention surface. Such varying retention force profiles can be beneficial when seeking to retain samples which have different consistencies across their whole.

Optionally the planar retention surface includes one or more areas having a reduced cross-sectional thickness.

The inclusion of one or more areas of reduced cross-sectional thickness provides for further tuning of the retention force profile applied across the retention surface.

In another preferred embodiment of the invention the retention element is formed from a resiliently deformable material.

Forming the whole of the retention element from a resiliently deformable material necessarily ensures that the entire distal end thereof is resiliently deformable, but additionally facilitates coupling of the retention element with the insert body, and simplifies manufacture of the retention element by allowing it to be made as a single component.

In another preferred embodiment of the invention the insert body includes at least one fluid conduit arranged in communication with the distal end of the retention element.

The provision of one or more such fluid conduits in the insert body desirably facilitates the selective introduction and removal of a liquid to/from contact with a sample retained by the distal end.

According to a second aspect of the invention there if provided a cell culture retaining insert assembly comprising a plurality of cell culture retaining inserts as described herein above fixedly secured to one another.

Such a cell culture retaining insert assembly is particularly well adapted to retaining a respective three-dimensional biological sample as desired in each of a plurality of wells within a cell culture vessel.

Optionally the plurality of cell culture retaining inserts have respective insert bodies that are integrally formed with one another.

Integrally forming the insert bodies of multiple cell culture retaining inserts with one another, e.g. as a single moulding, desirably simplifies the securing of such cell culture retaining inserts to one another, and therefore readily permits the manufacture of the cell culture retaining insert assembly of the invention.

According to a third aspect of the invention there is provided a cell culture positioning insert, for positioning a three-dimensional biological sample in a cell culture vessel, the cell culture positioning insert comprising a rigid insert body having:
at least one engagement formation to in-use removably secure the cell culture positioning insert to a cell culture vessel;
a tapered positioning conduit extending therethrough to a distal end thereof positionable in-use proximate to a well bottom in a cell culture vessel, the tapered positioning conduit in-use urging a three-dimensional biological sample placed therein towards a desired position in the well bottom of the cell culture vessel, and
at least one fluid conduit extending therethrough to arrange the positioning conduit in fluid communication with an exterior of the insert body.

Including at least one fluid conduit to arrange the positioning conduit in fluid communication with an exterior of the insert body facilitates the selective introduction and removal of a liquid to/from contact with a sample desirably positioned by the cell culture positioning insert within a well of a cell culture vessel.

According to a fourth aspect of the invention there is provided a cell culture positioning insert assembly comprising a plurality of cell culture positioning inserts as described hereinabove fixedly secured to one another.

Such a cell culture positioning insert assembly is particularly well adapted to positioning a respective three-dimensional biological sample as desired in each of a plurality of wells within a cell culture vessel.

Preferably the plurality of cell culture positioning inserts have respective insert bodies that are integrally formed with one another.

Integrally forming the insert bodies of multiple cell culture positioning inserts with one another, e.g. as a single moulding, again desirably simplifies the securing of such cell culture positioning inserts to one another, and therefore readily permits the manufacture of the cell culture positioning insert assembly of the invention.

According to a fifth aspect of the invention there is provided a cellular assay comprising a cell culture vessel having at least one well, the or each well having associated therewith a cell culture retaining insert as described hereinabove, the or each respective cell culture retaining insert being removably secured to the cell culture vessel.

Preferably in a cellular assay comprising a cell culture vessel having a plurality of wells, the plurality of wells have associated therewith a cell culture retaining insert assembly as described hereinabove.

Optionally the cellular assay further includes a cell culture positioning insert as described hereinabove interposed between the or each well and a corresponding cell culture retaining insert.

A cellular assay comprising a cell culture vessel having a plurality of wells, may have a cell culture positioning insert assembly as described hereinabove interposed between the plurality of wells and respective cell culture retaining inserts.

Providing such cellular assays extends the benefits associated with the cell culture retaining insert, the cell culture positioning insert, and the associated insert assemblies of the invention to cellular assays more generally.

It will be appreciated that the use of the terms "first" and "second", and the like, in this patent specification is merely intended to help distinguish between similar features, and is not intended to indicate the relative importance of one feature over another feature, unless otherwise specified.

Within the scope of this application it is expressly intended that the various aspects, embodiments, examples and alternatives set out in the preceding paragraphs, and the claims and/or the following description and drawings, and in particular the individual features thereof, may be taken independently or in any combination. That is, all embodiments and all features of any embodiment can be combined in any way and/or combination, unless such features are incompatible. The applicant reserves the right to change any originally filed claim or file any new claim accordingly, including the right to amend any originally filed claim to depend from and/or incorporate any feature of any other claim although not originally claimed in that manner.

There now follows a brief description of preferred embodiments of the invention, by way of non-limiting examples, with reference being made to the following figures in which:
Figure 1 shows a perspective view of a cell culture retaining insert according to a first embodiment of the invention;
Figure 2 shows a cross-sectional view of the cell culture retaining insert shown in Figure 1;
Figure 3 shows a perspective view of a cell culture retaining insert according to a second embodiment of the invention;
Figure 4 shows an enlarged perspective view of a distal end of a first retention element that may form a part of the cell culture retaining insert shown in Figure 3;
Figure 5 shows a cross-sectional view of the distal end of Figure 4 in a first use mode;
Figure 6 shows an enlarged, cross-sectional view of a distal end of a second retention element that may form a part of the cell culture retaining insert shown in Figure 3, in a second use mode;
Figure 7 shows an enlarged perspective view of a distal end of a third retention element that may form a part of the cell culture retaining insert shown in Figure 3;
Figure 8(a) shows a perspective, cross-sectional view of a cell culture retaining insert according to a third embodiment of the invention;
Figure 8(b) shows an enlarged perspective view of a retention element forming a part of the cell culture retaining insert shown in Figure 8(a);
Figure 9 shows a perspective view of a cell culture positioning insert according to a fourth embodiment of the invention;
Figure 10 shows a perspective view of a cell culture position insert assembly according to a fifth embodiment of the invention;
Figure 11 shows a perspective view of a portion of a cellular assay according to a sixth embodiment of the invention; and
Figures 12(a) to 12(c) show schematic perspective views of cellular assays according to further embodiments of the invention.

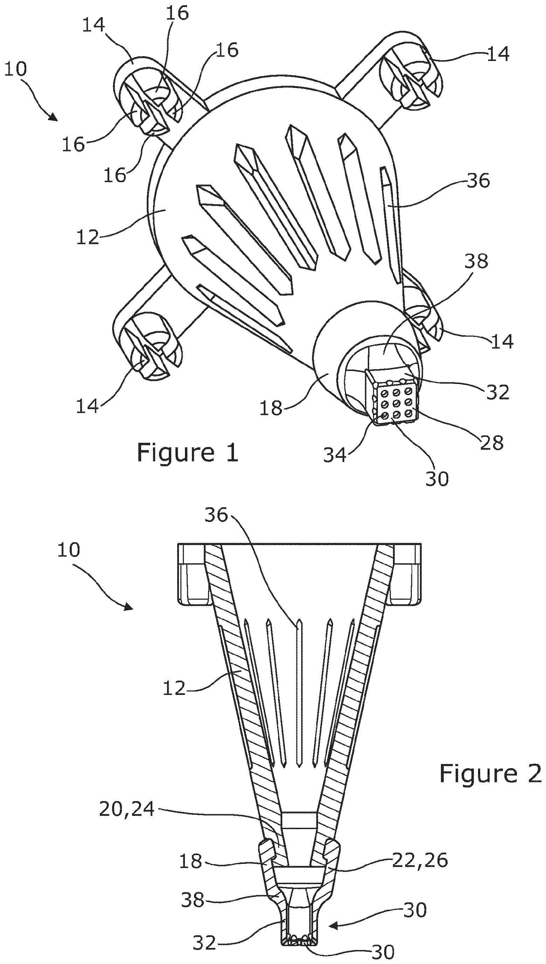

A cell culture retaining insert according to a first embodiment of the invention is designated generally by reference numeral 10, as shown in Figure 1.

The first cell culture retaining insert 10 includes a rigid insert body 12 which has four engagement formations 14 which, in use, removably secure the first retaining insert 10 to a cell culture vessel (not shown), such as an assay tray. Other embodiments of the invention may include fewer than or more than four engagement formations.

In the embodiment shown, each engagement formation 14 includes four moveable legs 16 which cooperate with a complementary receiving formation in an associated cell culture vessel to provide the required selective securing of the first retaining insert 10 to such a cell culture vessel. Again, in other embodiments of the invention one or more of the engagement formations may include fewer than or more than four legs, and indeed may include one or more engagement formations of a different configuration, such one or more different engagement formations being configured to cooperate with similarly different receiving formations arranged in a corresponding cell culture vessel. Returning to the first cell culture retaining insert 10 shown, it also includes a retention element 18 that is coupled with the insert body 12.

As shown in Figure 2, the retention element 18 may be coupled with the insert body 12 via mutually co-operable coupling formations 20, 24, such as a ring formation 24 on the insert body 12 and complementary gripping formation 26 on the retention element 18. Other mutually co-operation coupling formations are also possible, however.

Meanwhile, the retention element 18 has distal end 28, i.e. a furthest most part, which, in use, is positionable proximate to a well bottom in a cell culture vessel.

In the embodiment shown, the distal end 28 is formed of a planar retention surface 30, i.e. a retention surface 30, or planar retention portion, which has a substantially uniform thickness thereacross, which has four retention walls 32 that depend therefrom. Other embodiments (not shown) may include fewer than or greater than four retention walls, and still further embodiments may include retention walls interconnected by interconnecting walls which may or may not be curved interconnecting walls.

The entire distal end 28 is resiliently deformable, i.e. the whole of the planar retention surface 30 presented, in use, to a well bottom, and each of the retention walls 32 are all resiliently deformable, although in some embodiments only the whole of the planar retention surface, i.e. only the whole of the planar retention portion, need be resiliently deformable. More particularly, in the embodiment shown the whole of the retention element 18 is formed from a resiliently deformable material so as to imbue the entire distal end 28 with the desired resilient deformability. One example of such a resiliently deformable material is an elastomer such as silicone or a silicone rubber, although other suitable materials may be used.

In addition to the foregoing, the retention surface 30 includes a plurality of conduits 34, and more particularly nine conduits 34 in the embodiment shown, although this may vary in other embodiments. Such conduits 34 permit the passage of fluid therethrough as well as allowing a user, e.g. a lab technician or other scientist, to visually inspect or observe a biological sample through the retention surface 30.

Also, the insert body 12 includes a plurality of fluid conduits 36 that are arranged in communication with the distal end 28 of the retention element 18, and more particularly in fluid communication with the planar retention surface 30 and the conduits 34 extending therethrough. Such a combination of conduits 34, 36 readily permits the selective introduction and removal of a liquid to/from contact with a sample retained by the distal end 28.

Additionally, each of the retention walls 32 includes a tapered portion 38 which allows the retention surface 30 to be smaller than the overall diameter (or in other embodiments, other cross-sectional shapes) of the retention element 18.

The planar retention surface 30, i.e. planar retention portion, is also substantially flat. In other embodiments of the invention (not shown), the planar retention surface may include one or more areas that have a reduced cross-sectional thickness.

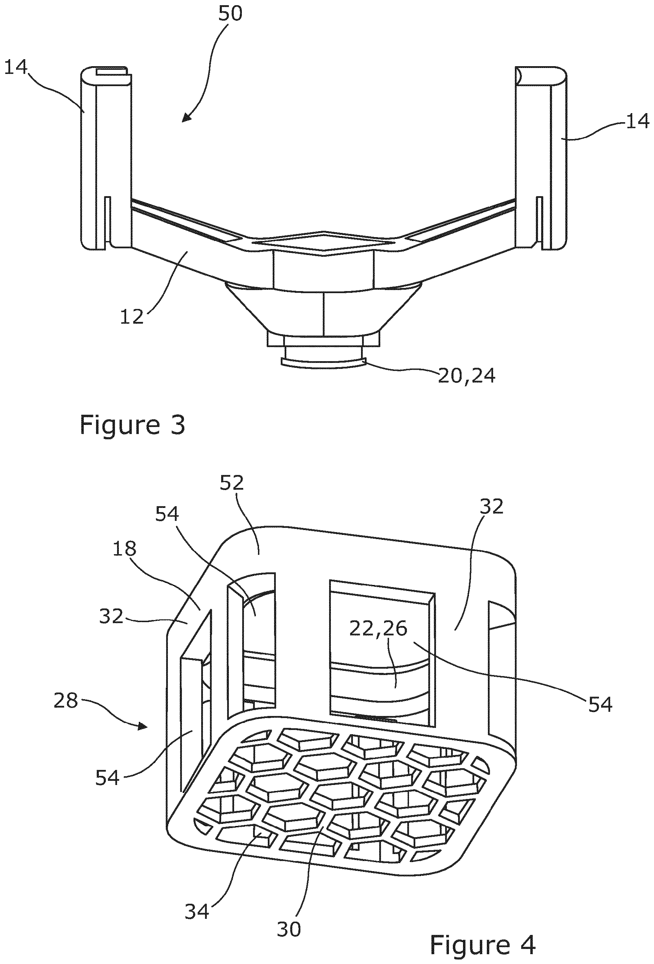

The insert body 12 of a cell culture retaining insert 50 according to a second embodiment of the invention is shown in Figure 3.

The second cell culture retaining insert 50 is similar to the first cell culture retaining insert 10 and, as indicated above, like features share the same reference numerals. The insert body 12 of the second retaining insert 50 does, however, include only two engagement formations 14, and they are configured differently to those of the first retaining insert 10.

In addition, as shown in Figure 4, the retention element 18 of the second retaining insert 50 differs from the retention element 18 of the first retaining insert 10.

More particularly, the retention element 18 of the second retaining insert 50 has a planar retention surface 30, i.e. a planar retention portion, with a plurality of substantially hexagonally-shaped conduits 34 passing therethrough (although other shaped conduits may also be used). Also the coupling formation 22, i.e. gripping formation 26 is formed immediately adjacent to the retention walls 32 (i.e. rather than being spaced from the retention walls 32, as in the first retaining insert 10), those retention walls 32 are interconnected by curved interconnecting walls 52, and all of the retentions walls 32 and interconnecting walls 52 have a further fluid conduit 54 passing therethrough.

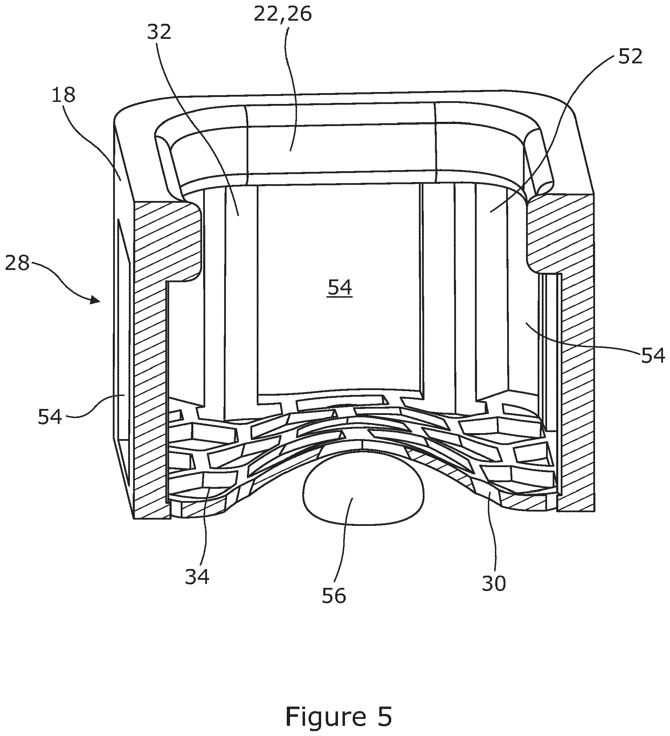

Figure 5 shows how, in a first use mode, the substantially flat planar retention surface 30, i.e. the planar retention portion, of the distal end 28 of the retention element 18, i.e. of the second retaining insert 50, deforms to accommodate a three-dimensional biological sample 56, while also urging the sample 56 into abutment with a well bottom (not shown) of a well within a cell culture vessel, and thereby retaining the sample 56 in a desired position within the well.

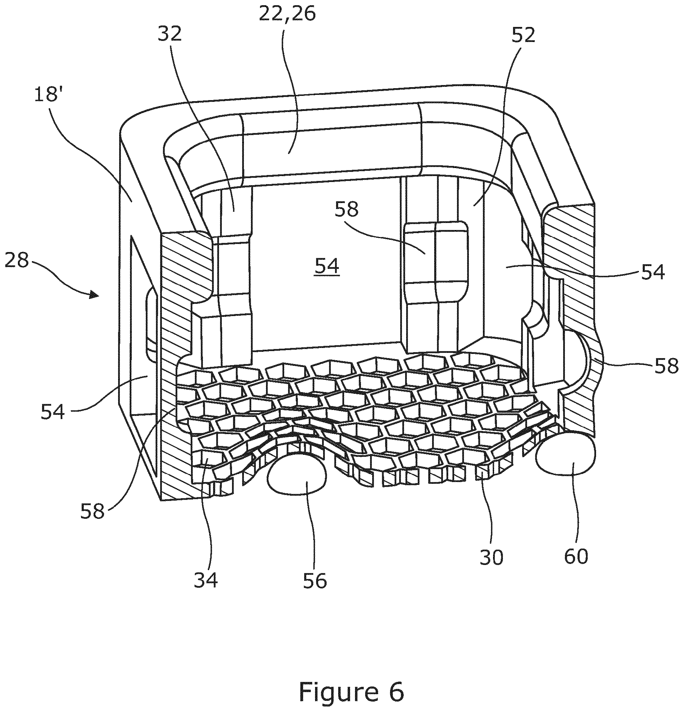

Meanwhile, Figure 6 shows a distal end 28 of a second retention element 18' that may form a part of the second cell culture retaining insert 50 shown in Figure 3.

The second retention element 18' is very similar to the first retention element 18 shown in Figures 4 and 5 but the plurality of substantially hexagonally-shaped conduits 34 passing therethrough differ in size and spacing to those in the first retention element 18.

In that regard, the size and shape of the conduits 34, as well as the distance between them, can be tuned according to two parameters, i.e. (i) the size of the biological sample to be retained; and (ii) the percentage of exposed surface of such a sample required for liquid exchange and/or inspection. For example, if the sample is 1mm in diameter (i.e. is an organoid), or is 200-300mm in diameter (i.e. is a spheroid), the size of the conduit 34 will need to be chosen to avoid the biological sample simply passing therethrough. Similarly, the distance between the conduits 34 can be determined by the percentage of exposed surface, which could be higher (e.g. 80%) for large samples (e.g., organoids, typically requiring more oxygen/media exchange) and smaller for small samples (e.g., spheroids). A large degree of flexibility in sizing the conduits 34 and the distance between them is possible within the limits imposed by the structural properties of the selected material from which the distal end is made.

Additionally, each of the retention walls 32 of the second retention element 18' has an area 58 of reduced cross-sectional thickness.

Meanwhile, as shown in Figure 6, in a second use mode, the substantially flat planar retention surface 30, i.e. planar retention portion, of the distal end 28 of the second retention element 18' deforms to accommodate a first three-dimensional biological sample 56 that is positioned away from the centre of a well bottom (not shown), while a retention wall 32 as well as part of the retention surface 30, i.e. the planar retention portion, deform to accommodate a second sample 60, lying in a still further different position on the well bottom. The retention surface 30 and the retention wall 32 again similarly acting together to urge the samples 56, 60 into abutment with the well bottom to thereby retain the samples 56, 60 in their respective positions within the well

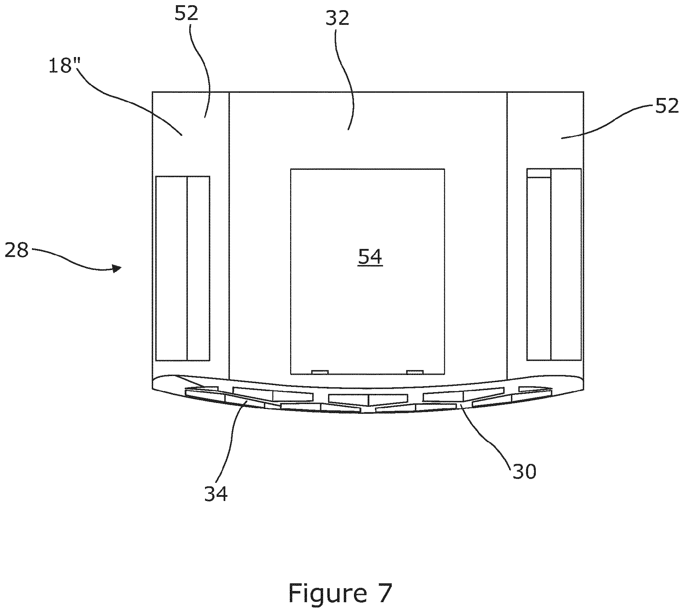

A third retention element 18" and its distal end 28, i.e. planar retention surface 30 and depending retention walls 32, which may form a part of the second cell culture retaining insert 50 shown in Figure 3, is shown in Figure 7.

The third retention element 18" is identical to the first retention element 18, save for the planar retention surface 30 thereof having a convex cross-sectional profile, i.e. save for having a planar retention surface 30 which bows outwards. Still further retention elements of other retaining insert embodiments may have a planar retention surface with a concave cross-sectional profile, i.e. a planar retention surface which bows inwards.

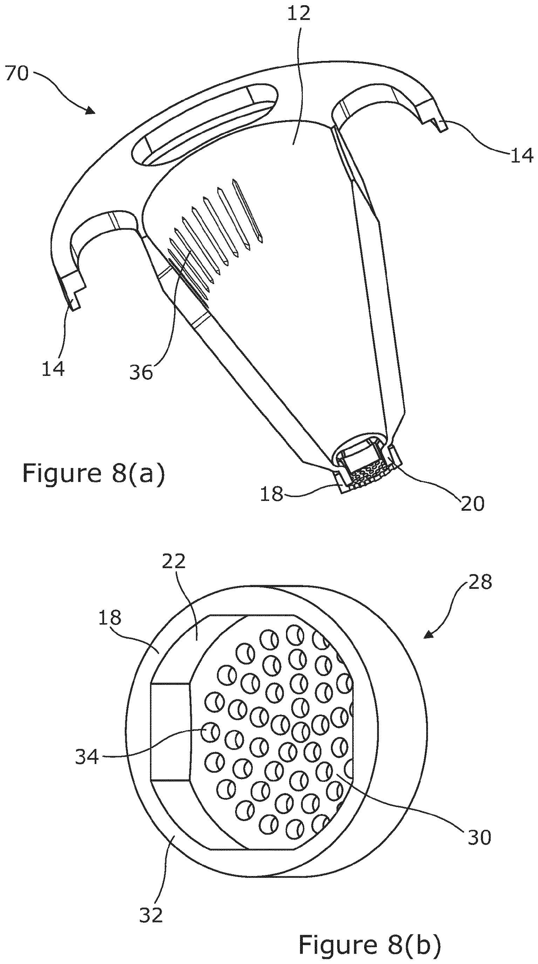

A cell culture retaining insert 70 according to a third embodiment of the invention is shown in cross-section in Figure 8(a), while an enlarged perspective view of the retention element 18 of the third retaining insert 70 is shown in Figure 8(b).

The third retaining insert 70 is most similar to the first retaining insert 10, and like features again share the same reference numerals.

The retention element 18 of the third retaining insert 70 differs however in that it includes eight retention walls 32 (rather than just four), and each of those retention walls 32 omits a tapered portion. Additionally, the coupling formation 22 is not configured as a defined gripping formation, but rather relies on a tight friction fit between the said retention walls 32 and the corresponding coupling formation 20 on the insert body 12 of the third retaining insert 70.

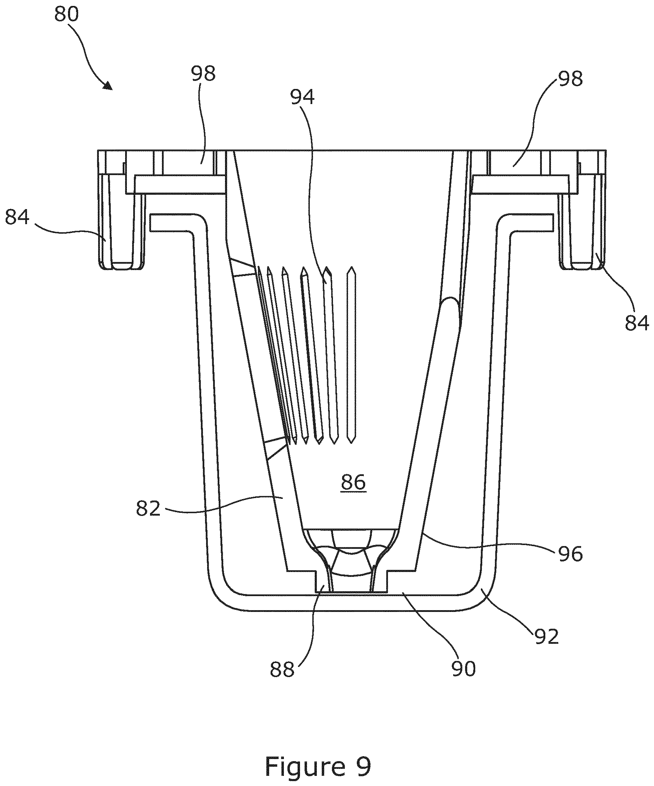

Figure 9 shows a cross-sectional, schematic view of a cell culture positioning insert 80 according to a fourth embodiment of the invention.

The positioning insert 80 similarly includes a rigid insert body 82 with four engagement formations 84 to, in use, removably secure the positioning insert 80 to a cell culture vessel.

The insert body 82 also includes a tapered positioning conduit 86 that extends through the insert body 82 to a distal end 88 thereof. The distal end 88 is positionable, in-use, proximate to a well bottom 90 of a well 92 in a cell culture vessel, as shown in Figure 9.

Additionally the insert body 82 includes a plurality of fluid conduits 94 extending therethrough so as to arrange the positioning conduit 86 in fluid communication with an exterior 96 of the insert body 82, and thereby permit the passage of fluid, e.g. an aqueous-based buffer, cell culture medium, or hydrogel, between the exterior 96 and the positioning conduit 86.

The insert body 82 also preferably includes a plurality of secondary fluid conduits 98 which desirably permit the introduction (or removal) of a fluid directly into the well 92, i.e. without having to pass between the positioning conduit 86 and exterior 96 of the insert body 82.

In use the tapered positioning conduit 86 urges a three-dimension biological sample 56, 60 towards a desired position in the well bottom 90 of the cell culture vessel, as shown in Figure 9.

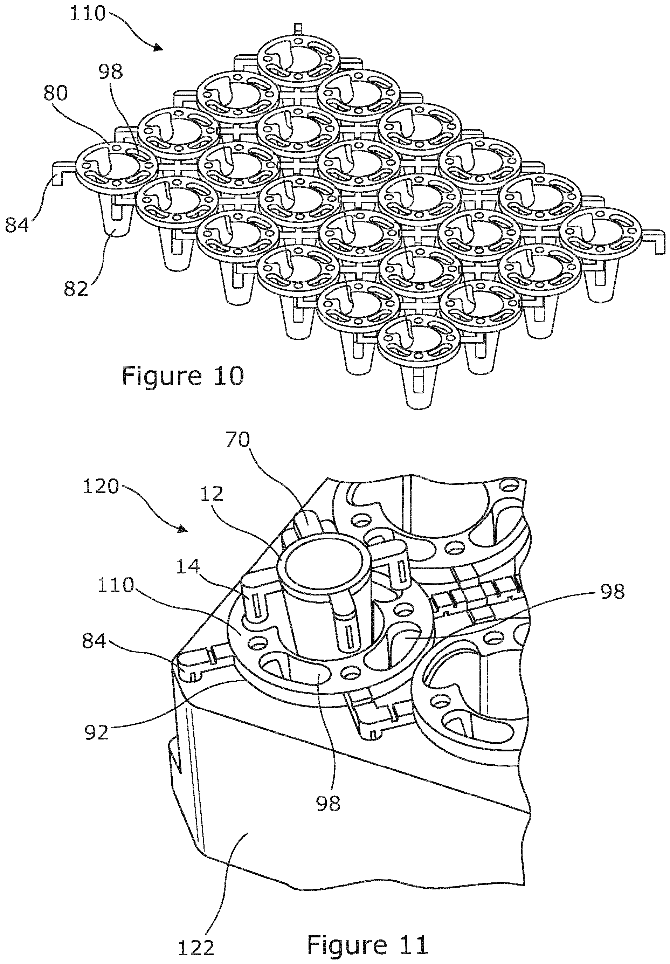

A further embodiment of the invention, in the form of a cell culture positioning insert assembly 110, is shown in Figure 10.

The positioning insert assembly 110 of the invention includes a plurality of, e.g. 24, cell culture positioning inserts 80 that are fixedly secured together. More particularly, in the embodiment shown the positioning inserts 80 have respective insert bodies 82 that are integrally formed, i.e. integrally moulded, with one another.

Other embodiments of cell culture positioning insert assemblies may have fewer than or more than 24 cell culture positioning inserts fixedly secured, e.g. integrally formed, to one another. Still further such positioning insert assemblies may have respective insert bodies that are integrally formed with one another in a different manner than moulding.

Figure 11 shows a portion of a cellular assay 120 according to a still further embodiment of the invention.

The first cellular assay includes a cell culture vessel 122 with a plurality of wells 92 (only three of which are shown). A cell culture retaining insert, e.g. the third retaining insert 70 is associated with each well 92 (only one third retaining insert 70 being shown), and is removably secured to the cell culture vessel 122.

More particularly, the cell culture positioning insert assembly 110 described above is interposed between the third retaining insert 70 and the plurality of wells 92, i.e. the cell culture vessel 122, and more particularly still, the positioning insert assembly 110 is removably secured to the cell culture vessel 122, e.g. via respective engagement formations 84 thereof, and the third retaining insert 70 is, in turn, removably secured to the positioning insert assembly 110 via the respective engagement formations 14 of the third retaining insert 70.

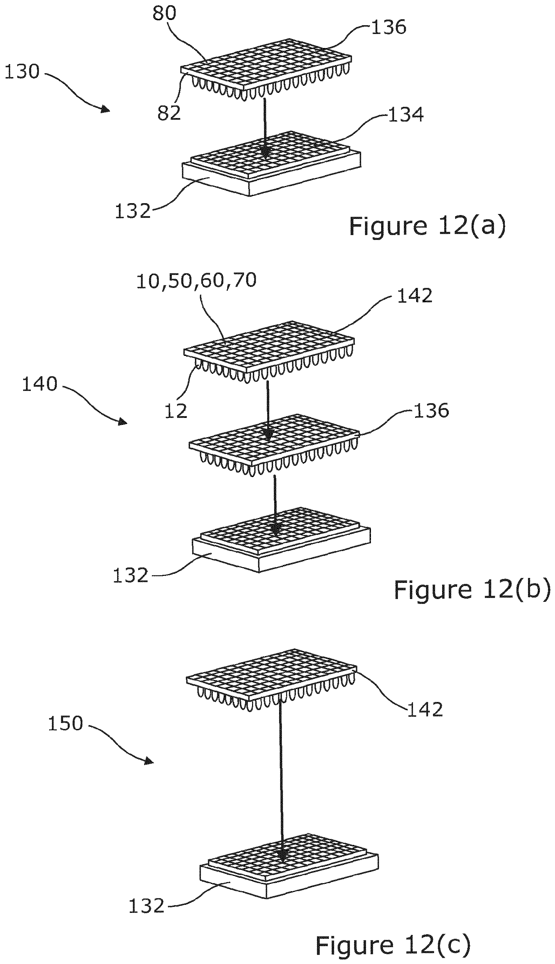

Figures 12(a), 12(b) and 12(c) show second, third and fourth cellular assays 130, 140, 150 according to further embodiments of the invention.

The second cellular assay 130 includes a multiwell cell culture vessel 132, e.g. a cell culture vessel having 72 wells 134, along with a cell culture positioning insert assembly 136 having an identical number, i.e. 72, of cell culture positioning inserts 80 that are fixedly secured to one another, e.g. have respective insert bodies 82 that are integrally moulded with one another. The positioning insert assembly 136 is removably secured to the cell culture vessel 132.

The third cellular assay 140 similarly includes the aforementioned multiwell cell culture vessel 132 and the positioning insert assembly 136, as well as a cell culture retaining insert assembly 142. The retaining insert assembly 142 has an identical number, i.e. 72, cell culture retaining inserts (i.e. any of the first, second, or third retaining inserts 10; 50; 70) that are similarly fixedly secured to one another, e.g. have respective insert bodies 12 that are integrally formed, for example, moulded, with one another. The positioning insert assembly 136 is again removably secured to the cell culture vessel 132, while the retaining insert assembly 142 is removably secured to the positioning insert assembly 136 and thereby to the cell culture vessel 132.

The fourth cellular assay 150 is similar to each of the second and third cellular assays 130, 140 but omits a positioning insert assembly, and so the corresponding retaining insert assembly 142 is instead removably secured directly with the cell culture vessel 132.

## Claims

1. A cell culture retaining insert, for retaining a three-dimensional biological sample in a cell culture vessel, the cell culture retaining insert comprising:
a rigid insert body having at least one engagement formation to in-use removably secure the cell culture retaining insert to a cell culture vessel; and
a retention element coupled with the insert body, the retention element having a distal end positionable in-use proximate to a well bottom in a cell culture vessel, the entire distal end being resiliently deformable.

2. A cell culture retaining insert according to Claim 1 wherein the distal end of the retention element is or includes a planar retention surface having one or more retention walls depending therefrom.

3. A cell culture retaining insert according to Claim 2 wherein the planar retention surface includes a plurality of conduits passing therethrough.

4. A cell culture retaining insert according to Claim 2 or Claim 3 wherein at least one retention wall includes one or more fluid conduits passing therethrough.

5. A cell culture retaining insert according to any of Claims 2 to 4 wherein at least one retention wall includes a tapered portion.

6. A cell culture retaining insert according to any of Claims 2 to 5 wherein at least one retention wall includes one or more areas having a reduced cross-sectional thickness.

7. A cell culture retaining insert according to any of Claims 2 to 6 wherein the planar retention surface is substantially flat.

8. A cell culture retaining insert according to any of Claims 2 to 6 wherein the planar retention surface has a convex or a concave cross-sectional profile.

9. A cell culture retaining insert according to Claim 7 or Claim 8 wherein the planar retention surface includes one or more areas having a reduced cross-sectional thickness.

10. A cell culture retaining insert according to any preceding claim wherein the retention element is formed from a resiliently deformable material.

11. A cell culture retaining insert according to any preceding claim wherein the insert body includes at least one fluid conduit arranged in communication with the distal end of the retention element.

12. A cell culture retaining insert assembly comprising a plurality of cell culture retaining inserts according to any preceding claim fixedly secured to one another.

13. A cell culture retaining insert assembly according to Claim 12 wherein the plurality of cell culture retaining inserts have respective insert bodies that are integrally formed with one another.

14. A cell culture positioning insert, for positioning a three-dimensional biological sample in a cell culture vessel, the cell culture positioning insert comprising a rigid insert body having:
at least one engagement formation to in-use removably secure the cell culture positioning insert to a cell culture vessel;
a tapered positioning conduit extending therethrough to a distal end thereof positionable in-use proximate to a well bottom in a cell culture vessel, the tapered positioning conduit in-use urging a three-dimensional biological sample placed therein towards a desired position in the well bottom of the cell culture vessel, and
at least one fluid conduit extending therethrough to arrange the positioning conduit in fluid communication with an exterior of the insert body.

15. A cell culture positioning insert assembly comprising a plurality of cell culture positioning inserts according to Claim 14 fixedly secured to one another.

16. A cell culture positioning insert assembly according to Claim 15 wherein the plurality of cell culture positioning inserts have respective insert bodies that are integrally formed with one another.

17. A cellular assay comprising a cell culture vessel having at least one well, the or each well having associated therewith a cell culture retaining insert according to any of Claims 1 to 11, the or each respective cell culture retaining insert being removably secured to the cell culture vessel.

18. A cellular assay according to Claim 17 comprising a cell culture vessel having a plurality of wells, the plurality of wells having associated therewith a cell culture retaining insert assembly according to Claim 12 or Claim 13.

19. A cellular assay according to Claim 17 or Claim 18 further including a cell culture positioning insert according to Claim 14 interposed between the or each well and a corresponding cell culture retaining insert.

20. A cellular assay according to Claim 19 comprising a cell culture vessel having a plurality of wells, the cellular assay having a cell culture positioning insert assembly according to Claim 15 interposed between the plurality of wells and respective cell culture retaining inserts.
